# EUROPEAN PATENT SPECIFICATION

(11) **EP 1 108 724 B1**
(45) Date of publication and mention of the grant of the patent: **19.09.2007**
(21) Application number: 01103093.9
(22) Date of filing: 23.12.1996
(51) Int. Cl.: C07H 19/06, C07H 19/16, C07H 21/02, C12N 9/00, A61K 31/70

(54) **Synthesis of methoxy nucleosides and enzymatic nucleic acid molecules**
Synthese von Methoxynukleoside und enzymatische Nukleisäure Moleküle
Synthèse de methoxy nucléosides et de molécules d'acide nucléique enzymatique

(30) Priority: 16.01.1996 US 585682; 13.02.1996 US 600429; 16.04.1996 US 632882
(43) Date of publication of application: 20.06.2001
(62) Divisional of application: 96944523.8
(73) Proprietor: SIRNA THERAPEUTICS, INC., Boulder, CO 80301 (US)
(72) Inventor: Wincott, Francine c/o Eyetech Pharmaceuticals, Inc., 18th Floor New York NY 10018 (US); Beigelmann, Leonid, Longmont, CO 80503 (US); Matulic-Adamic, Jasenka, Boulder, CO 80303 (US); Usman, Nassim, Boulder, CO 80304 (US); Haeberli, Peter, Berthoud,CO 80513 (US); Karpeisky, Alexander, Boulder, CO 80301 (US); Sweedler, David, Louisville, CO 80027 (US); Jarvis, Thale, Boulder, Colorado (US); Direnzo, Anthony, Boulder, CO 80303 (US)
(74) Representative: Viering, Jentschura & Partner

(56) References cited:
- BEIGELMAN L. ET AL: "Alternative routes to the synthesis of 2'-O-Me nucleosides" NUCLEOSIDES & NUCLEOTIDES., vol. 14, no. 3-5, 1995, pages 241-425, XP002033737 INC US
- T. AZUMA, K. ISONO: "Transnucleosidation: An improved method for transglycosylation from pyrimidines to purines" CHEM. PHARM. BULL., vol. 25, 1977, pages 3347-3353, XP002206557
- ROBINS M.J. ET AL: "Nucleic Acid Related Compounds. 12. The Facile and High-Yield Stannous Chloride Catalyzed Monomethylation of the Cis-Glycol System of Nucleosides by Diazomethane" JOURNAL OF ORGANIC CHEMISTRY, vol. 39, no. 13, 1974, pages 1891-1899, XP002033777 EASTON US
- YAMAUCHI K ET AL: "SELECTIVE 2'-O-METHYLATION OF PYRIMIDINE-RIBONUCLEOSIDES BY TRIMETHYLSULFONIUM HYDROXIDE IN THE PRESENCE OF MG2+ AND CA2+ IONS" BULLETIN OF THE CHEMICAL SOCIETY OF JAPAN, vol. 59, no. 9, 1 September 1986 (1986-09-01), pages 2947-2949, XP000571563

## Description

### Background of the Invention

This invention relates to the chemical synthesis of 2'-*O*-methyl, nucleosides.

In one aspect the invention features chemical synthesis of 2'-*O*-methyl nucleosides. The following is a brief description of synthesis of methoxy nucleosides. This summary is not meant to be complete but is provided only for understanding of the invention that follows. This summary is not an admission that all of the work described below is prior art to the claimed invention.

Sugar modifications, such as 2'-*O*-methyl, have been discovered in a variety of naturally occurring RNA (*e.g.,* tRNA, mRNA, rRNA; reviewed by Hall, 1971 The Modified Nucleosides in Nucleic Acids, Columbia University Press, New York; Limbach et al., 1994 Nucleic Acids Res. 22, 2183). In an attempt to understand the biological significance, structural and thermodynamic properties, and nuclease resistance of these sugar modifications in nucleic acids, several investigators have chemically synthesized nucleosides, nucleotides and phosphoramidites of various sugar modifications and incorporated them into oligonucleotides. There are several reports in the literature describing the synthesis of 2'-*O*-methyl nucleosides, 2'-*O*-methyl nucleotides, 2'-*O*-methyl phosphoramidites and oligonucleotides containing 2'-*O*-methyl substitutions (Broom and Robins, 1965 J. Am. Chem. Soc. 87, 1145; Martin et al., 1968 Biochemistry, 7, 1406; Robins et al., 1974 J. Org. Chem. 39, 1891; Inoue et al., 1987 Nucleic Acids Res. 15, 6131; Cotten et al., 1991 Nucleic Acids Res. 19, 2629; Andrews et al., 1994 J. Heterocyclic Chem. 31, 765; Beigelman et al., 1995 Nucleosides & Nucleotides 14, 421; Sproat et al., 1990 Nucleic Acids Res. 18, 41).

Broom and Robins, 1965 J. Am. Chem. Soc. 87, 1145 and Martin et al., 1968 Biochemistry, 7, 1406, describe the synthesis of 2'-*O*-methyl ribonucleotides involving mono-methylation of a 2',3'-cis-diol system of a ribonucleoside with diazomethane. This procedure gives rise to a mixture of 2'- and 3'-*O*-methyl nucleosides in 20-40% combined yield. The two isomers are then separated by ion-exchange chromatography.

Robins et al., 1974 J. Org. Chem. 39, 1891, describe the treatment of a methanolic solution of uridine with diazomethane (in glyme) in the presence of stannous chloride dihydrate (in methanol) to synthesize 2'-*O*-methyluridine (58% yield). This reaction also yielded a significant fraction (28%) of 3'-*O-*methyluridine which is purified away from the 2'-*O*-methyl form by chromatography.

Inoue, Japanese Patent Publication No: 61291595 and Inoue et al., 1987 Nucleic Acids Res. 15, 613, describe a process for the synthesis of 2'-*O-*methyl ribonucleosides involving alkylation of 3',5'-*O-*(tetraisopropyldisiloxane-1, 3-diyl) (TIPDS)-ribonucleosides with methyl iodide. Inoue *et al.,* state that (page 6133, second main paragraph):
Treatment of 3',5'-*O*-TIDPS-uridine (1) with benzoyl chloride...in N,N-dimethylacetamide in the presence of triethylamine... selectively gave the N³-benzoylated derivative (2) in 70.5% yield. Then, 2 was treated, with CH₃ₗ...in benzene in the presence of Ag₂O...at 40°C overnight to give the N³-benzoyl-2'-*O-*methyl derivative (3, 84.5%). Debenzoylation of 3 with dil. NH₄OH followed by removal of TIPDS group with 0.5N HCl afforded 2'-*O*-methyluridine...in 84% yield."

Srivastava and Roy, US Patent No. 5,214,135*,* describe the synthesis of 2'-*O*-methyl nucleosides using an approach similar to Inoue *et al., supra,* except that the reaction with methyl iodide/silver.oxide was carried out at 25°C for 24-46 hr with an 80-86% yield. This reaction, similar to the one described by Inoue *et al., supra,* also gave rise to the 3'-*O*-methyl isomer in 6-8% yield.

Parmentier et al., 1994 Tetrahedron 50, 5361, describe a convergent synthesis of 2'-*O*-methyl uridine. This procedure uses a multi-step process involving- 'a facile obtention of the 2'-*O*-methyl sugar synthon using totally selective and efficient methylation conditions;...a stereoselective high-yield condensation with an uracil derivative, yielding the desired β-form with a satisfactory anomeric excess. (page 5361, fifth paragraph).

Chanteloup and Thuong, 1994 Tetrahedron Letters 35, 877, describe synthesis of 2'-*O*-alkyl ribonucleosides using trichloroacetimidate D-ribofuranosides as ribosyl donors. They state in the abstract on page 877-
Trichloroacetimidate-2-*O*-alkyl-3,5-*O*-TIPS-β-D-ribofuranoside glycosylates silylated nucleobases in a fast high-yielding and stereoselective reaction promoted by trimethylsilyl trifluoromethanesulfonate. This method has been applied to the synthesis of 2'-*O*-alkyl ribonucleosides further transformed to building blocks ready for oligo(2'-*O*-alkyl)ribonucleotide construction.

Beigelman *et al.,* 1995 *Nucleosides & Nucleotides* 14, 421, describe three different approaches to the synthesis of 2'-*O*-methyl nucleosides. They state that-

### Method 1:

"Retrosynthetic analysis showed that 3-*O*-alkylated derivatives of 1,2:5,6-di-*O*-isopropylidene(IP)-α-D-allofuranose (1) could be transformed to the related 2'-*O-*alkyl ribofuranosides by selective degradation of the C1-C2 bond with subsequent cyclization of the generated C2-formyl group to the C5-OH." (Page 421, third paragraph)

### Method 2:

"The 3'-*O*-TBDMS-derivatives of protected ribonucleosides are byproducts obtained during the preparation of 2'-*O*-TBDMS derivatives - key building blocks in oligoribonucleotide synthesis. At the same time, 3'-*O*-TBDMS-isomers could be useful starting compounds in the preparation of 2'-*O*-methyl-3'-*O*-phosphoramidites. We explored this possibility on cytidine derivative 14. Reaction of 3'-*O*-TBDMS-5'-*O-*DMT-N⁴-*i*-Bu-cytidine (14) with Ag₂O-CH₃l using a modified method of Ohtsuka *et al. (supra)* yielded 3'-*O*-TBDMS-5'-*O*-DMT-N⁴-*i*-Bu-2'-*O*-methyl cytidine (**15**) in 26% yield. The 2'-*O*-TBDMS isomer **16** was also obtained (22% yield) along with the starting 3'-*O*-isomer (18%). When 2'-*O*-TBDMS-5'-*O*-DMT-N⁴-*i*-Bu-cytidine (**16**) was subjected to the same reaction conditions, the same mixture of products was obtained. These results show that under the above reaction conditions migration of the TBDMS group accompanies the methylation reaction and methylation takes place selectively at the 2'-OH position." (Page 422, second full paragraph)

### Method 3:

"Among different methods of indirect introduction of a methyl group, the use of 1-alkylthioalkyl intermediates seems to be the most promising. Although methods of synthesis of methylthiomethyl ethers of nucleosides and carbohydrates are well developed, their transformation into a methyl group sometimes requires additional steps. We were interested in the testing of more reactive methylthiophenyl ethers as precursors for methyl ethers. We found that methylthiophenyl ethers could be smoothly Introduced by treating appropriately protected nucleosides or carbohydrates with PhSMe/Bz₂O₂ in the presence of DMAP. Nucleoside **19** afforded methythiophenyl ether **20** in 65-70% yield, and α-ribofuranose **21** was transformed into β-turanose **22** in 60% yield. Different attempts to radically (Bu₃SnH, Bz₂O₂) reduce the thiophenyl group of furanose **22** were not successful, providing only starting material. However, under the same conditions, nucleoside **20** afforded 2'-*O*-Me.derivative **24** in 70% yield.

Haga et al., 1972 Carbohydrate Res. 21, 440 describe a "facile route" to the synthesis of 2- and 3-*O*-methyl-D-ribose from 3-*O*-methyl-D-allose.

Nair et al., 1982, Synthesis 8, 670, describes the modification of Nucleic acid bases via radical intermediates.

Leonard et al., 1992, Nucleosides & Nucleotides, 11, 1201, describe a method for the preparation of protected 2'-O-methylguanosine. This procedure is distinct from the one described in the instant invention.

Wagner et al., 1991, Nucleic Acids Res., 19, 5965, describes a method for alkylation of ribonucleosides.

Further, methods for the synthesis of enzymatic nucleic acid molcules.

Ribozymes are nucleic acid molecules having an enzymatic activity which is able to repeatedly cleave other separate RNA molecules in a nucleotide base sequence specific manner. Such enzymatic RNA molecules can be targeted to virtually any RNA transcript, and efficient cleavage achieved in vitro. Kim et al., 84 Proc. Natl. Acad. Sci, USA 8788, 1987; Haseloff and Gerlach, 334 Nature 585, 1988; Cech, 260 JAMA 3030, 1988; and Jefferies et al., 17 Nucleic Acids Research 1371, 1989.

Ribozymes act by first binding to a target RNA. Such binding occurs through the target RNA binding portion of a ribozyme which is held in close proximity to an enzymatic portion of the RNA which acts to cleave the target RNA. Thus, the ribozyme first recognizes and then binds a target RNA through complementary base-pairing, and once bound to the correct site, acts enzymatically to cut the target RNA. Strategic cleavage of such a target RNA will destroy its ability to direct synthesis of an encoded protein. After a ribozyme has bound and cleaved its RNA target it is released from that RNA to search for another target and can repeatedly bind and cleave new targets.

By "complementarity" is meant a nucleic acid that can form hydrogen bond(s) with other RNA sequences by either traditional Watson-Crick or other non-traditional types (for example, Hoogsteen type) of base-paired interactions.

Six basic varieties of naturally-occurring enzymatic RNAs are known presently. Each can catalyze the hydrolysis of RNA phosphodiester bonds in *trans* (and thus can cleave other RNA molecules) under physiological conditions. Table I summarizes some of the characteristics of these ribozymes. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The general structure of various ribozyme motifs is described by Draper et al. WO 93/23569, at pages 3-4 and in Usman et al., 95/06731 at pages 1-7 hereby incorporated by reference herein in its entirety (including drawings). Other motifs are also known in the art.

The enzymatic nature of a ribozyme is advantageous over other technologies, such as antisense technology (where a nucleic acid molecule simply binds to a nucleic acid target to block its translation) since the effective concentration of ribozyme necessary to effect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding, but also on the mechanism by which the molecule inhibits the expression of the RNA to which it binds. That is, the inhibition is caused by cleavage of the RNA target and so specificity is defined as the ratio of the rate of cleavage of the targeted RNA over the rate of cleavage of non-targeted RNA. This cleavage mechanism is dependent upon factors additional to those involved in base pairing. Thus, it is thought that the specificity of action of a ribozyme is greater than that of antisense oligonucleotide binding the same RNA site.

Jennings et al., US Patent No. 5, 298, 612 describe the use of non-nucleotides to assemble a hammerhead ribozyme lacking a stem II portion.

Draper et al., WO 93/23569 (PCT/US93/04020) describes synthesis of ribozymes in two parts in order to aid in the synthetic process (see, e.g., p. 40).

Usman et al., WO 95/06731, describe enzymatic nucleic acid molecules having non-nucteotides within their structure. Such non-nucleotides can be used in place of nucleotides to allow formation of an enzymatic nucleic acid.

The references cited above are distinct from the presently claimed invention since they do not disclose and/or contemplate the the methods used for the synthesis of methoxy nucleosides.

### Summary of the Invention

### Chemical synthesis of 2'-O-methyl, nucleosides

It has been postulated (Ueda, in Chemistry of Nucleosides and Nucleotides ed. L.B Townsend, v.1 Plenum Press 1988 pp. 1-95) that protonation of the N₃ atom of 2,2'-, 2,3' or 2,5'-anhydro pyrimidine nucleosides facilitates anhydro ring opening by different nucleophiles producing, in most cases, nucleoside analogs containing modifications in the carbohydrate portion of the nucleoside. Complexation of the N₃ atom of the above-mentioned anhydro derivatives with Lewis acids [*e.g*. B(OMe)₃] would provide the same effect directly, or in the case of methanolysis, complexation of the MeOH with Lewis acids would acidify the related proton leading to potential protonation of the N₃ atom of the above-mentioned anhydro derivatives. Applicant investigated methanolysis of 2,2'-, 2,3' or 2,5'-anhydro pyrimidine nucleosides in the presence of a Lewis acid, such as B(OMe)₃ and/or BF₃.MeOH. The reaction involving a 2,2'-anhydro-1(β-D-arabinofuranosyl) nucleoside, such as 2,2'-anhydro-1(β-D-arabinofuranosyl) uracil or 2,2'-anhydro-1 (β-D-arabinofuranosyl) cytosine, with B(OMe)₃ and/or BF₃.MEOH, results in the production of 2'-*O*-methyl nucleosides with a yield of about 90-100%.

By "Lewis Acid" is meant a substance that can accept an electron pair from a base. Examples of Lewis acids are, B(OCH₃)₃, BF₃, AlCl₃, and SO₃.

In one aspect, the invention features a process for the synthesis of a 2'-*O-*methyl adenosine nucleoside, comprising the step of contacting a solution of N⁴-acetyl-5',3'-di-*O*-acetyl-2'-*O*-methyl cytidine with a Lewis acid under conditions suitable for the formation of said nucleoside.

This invention features an improved and economical synthetic method for the preparation of 2'-*O*-methyl nucleosides in high yield. The method is not only cost efficient, but can be scaled up to several hundred gram quantities. The method generally utilizes inexpensive commercially available 2,2'-anhydro-1 (β-D-arabinofuranosyl)base,such as 2,2'-anhydro-1 (β-D-arabinofuranosyl)uracil or 2,2'-anhydro-1 (β-D-arabinofuranosyl)cytosine, as a starting material which is converted in a one or two step reaction sequence to 2'-*O*-methyl nucleosides with a yield of about 90-100%.

The 2'-*O*-methyl nucleosides can be used for chemical synthesis of nucleotides, nucleotide-tri-phosphates and/or phosphoramidites as a building block for selective incorporation into oligonucleotides. These oligonucleotides can be used as an antisense molecule, 2-5A antisense chimera, triplex molecule or as an enzymatic nucleic acid molecule. The oligonucleotides can also be used as probes or primers for synthesis and/or sequencing of RNA or DNA.

By "antisense" it is meant a non-enzymatic nucleic acid molecule that binds to target RNA by means of RNA-RNA or RNA-DNA or RNA-PNA (protein nucleic acid; Egholm et al., 1993 Nature 365, 566) interactions and alters the activity of the target RNA (for a review see Stein and Cheng, 1993 Science 261, 1004).

By "2-5A antisense chimera" it is meant, an antisense oligonucleotide containing a 5' phosphorylated 2'-5'-linked adenylate residues. These chimeras bind to target RNA in a sequence-specific manner and activate a cellular 2-5A-dependent ribonuclease which, in turn, cleaves the target RNA (Torrence et al., 1993 Proc. Natl. Acad. Sci. USA 90, 1300).

By "triplex DNA" it is meant an oligonucleotide that can bind to a doublestranded DNA in a sequence-specific manner to form a triple-strand helix. Formation of such triple helix structure has been shown to inhibit transcription of the targeted gene (Duval-Valentin et al., 1992 Proc. Natl. Acad. Sci. USA 89, 504).

By "enzymatic nucleic acid" it is meant a nucleic acid molecule capable of catalyzing reactions including, but not limited to, site-specific cleavage and/or ligation of other nucleic acid molecules, cleavage of peptide and amide bonds, and trans-splicing.

In preferred embodiments, the invention features a method for chemical synthesis of 2'-*O*-methyl nucleosides in which 2,2'-anhydro-1 (β-D-arabinofuranosyl) cytosine, 2.2'-anhydro-1(β-D-arabinofuranosyl) uracil, 2,3'-anhydro-1(β-D-arabinofuranosyl) uracil, or 2,3'-anhydro-1(β-D-arabinofuranosyl) cytidine is used as the starting material, and wherein said starting material is reacted with a Lewis acid.

### Description of the Preferred Embodiments

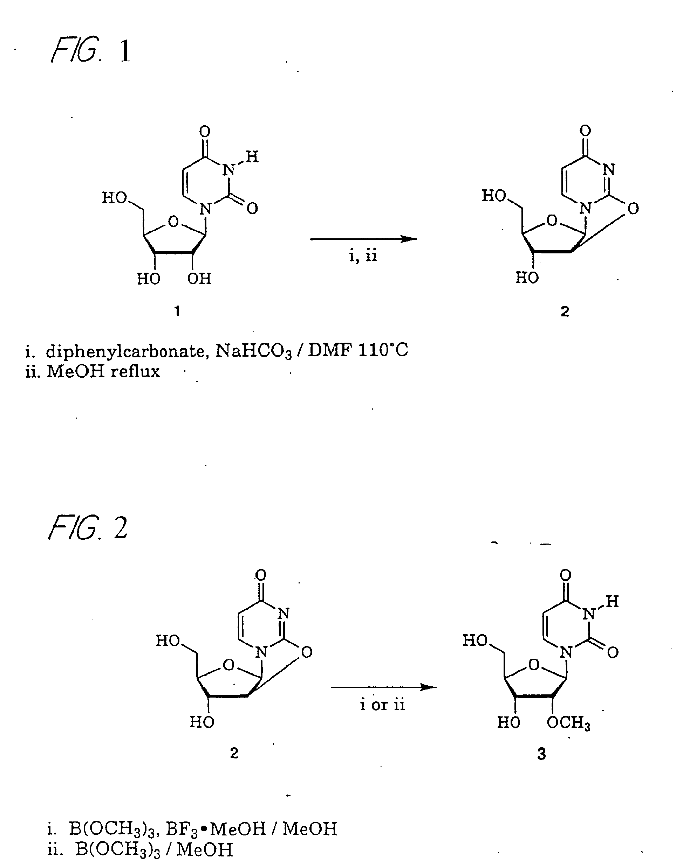

Figure 1 is a diagrammatic representation of a scheme involved in the synthesis of 2,2'-anhydro-1(β-D-arabinofuranosyl) uracil (**2**).
Figure 2 is a diagrammatic representation of a scheme involved in the synthesis of 2'-*O*-methyl uridine (**3**) by the method of this invention.

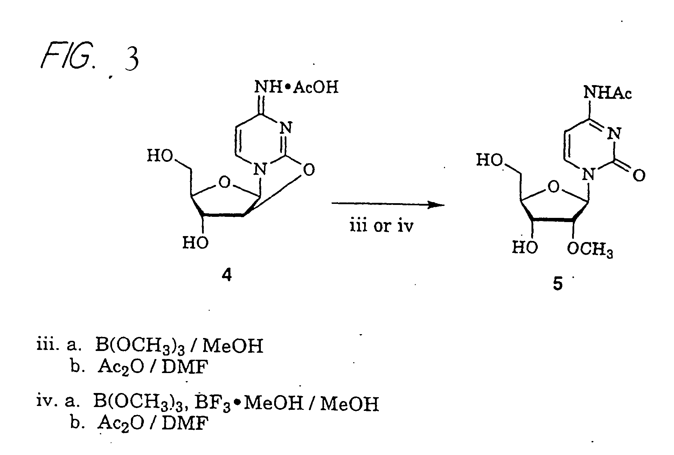

Figure 3 is a diagrammatic representation of a scheme involved in the synthesis of 2'-*O*-methyl cytidine (**5**) by the method of this invention.
Figure 4 shows NMR profile of 2'-*O*-methyl nucleosides. A) 2'-*O*-methyl Uridine nucleoside. B) 2'-*O*-methyl cytidine nucleoside.
Figure 5 is a diagrammatic representation of a scheme for the synthesis of 2'*-O*-methyl adenosine nucleoside.

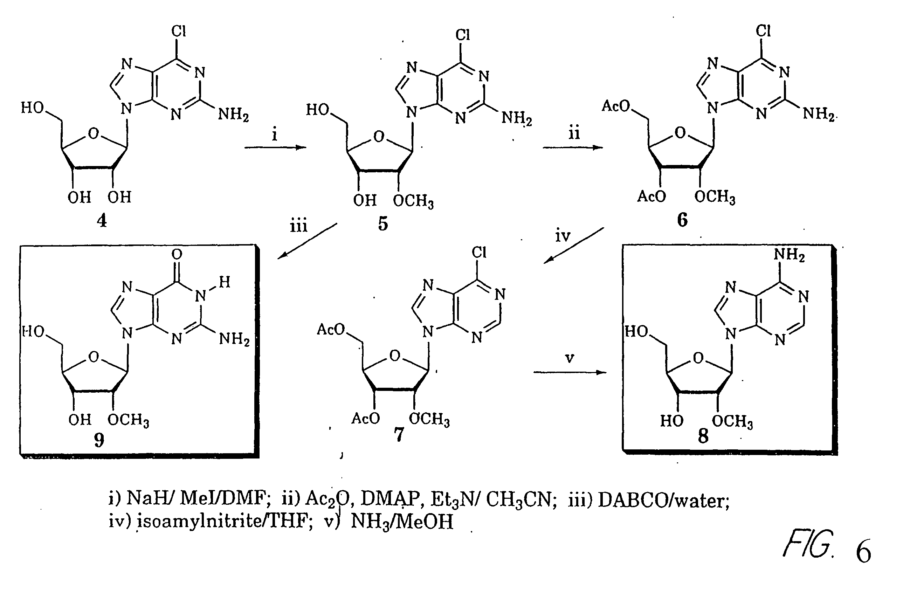

Figure 6 is a diagrammatic representation of a scheme for the synthesis of 2'-*O*-methyl adenosine and guanosine nucleosides.

### Examples

The following are non-limiting examples showing the synthesis of methoxy nucleosides.

### Example 1: Synthesis of 2,2'-anhydro-1(β-D-arabinofuranosyl) uracil (2)

2,2'-anhydro-1(β-D-arabinofuranosyl) uracil (**2**) can either be purchased from Sigma Chemicals or can be synthesized using the scheme described by Verheyden et al., 1971 (J. Org. Chem. 36, 250). Briefly, to an oven baked 1L 3-neck round bottom flask equipped with mechanical stirrer, reflux condenser, and positive pressure of argon, 200g (0.819 mol) of uridine (**1**) was added. The reaction was carried out in the presence of diphenylcarbonate (191.2 g, 0.9 mol), and DMF (300 ml). The resulting light yellow suspension was heated to 90°C at which time sodium bicarbonate (2.0 g) was added. The reaction mixture was then heated to 110°C for two hours during which time CO₂ evolved. Over this two hour period, the reaction mixture transformed from a slurry to a homogeneous solution and back to a slurry. Upon cooling to -10°C, the reaction mixture was filtered and the filter bed washed with ethanol and cold methanol. The filter bed was then suspended in methanol (500 ml) and heated to reflux for three hours. After cooling to -10°C, the reaction mixture was filtered. The filter bed was washed with cold methanol and dried to retrieve 2 as an off white solid (140g; 76%).

### Example 2: Synthesis of 2'-O-methyl uridine (3)

To an oven baked stainless steel bomb (300 ml), equipped with magnetic stirrer and purged with argon, 40 ml anhydrous methanol was added followed by the addition of 2,2'-anhydro-1-(β-D-arabinofuranosyl)uracil 2 (1.0 g, 4.42 mmol). To the resulting slurry, 5.0 ml trimethylborate (44.2 mmol) was added followed by the addition of boron trifluoride-methancl (50%) (1.5 ml, 8.84 mmol). The bomb was then sealed and heated in an oil bath at 130°C for 18 hours. Upon cooling, the resulting clear, slightly colored reaction mixture was evaporated *in vacuo* to yield a dark foam. The crude foam was dissolved in minimal methanol/dichloromethane and applied to a flash silica gel column. A gradient of 10-30% EtOH in dichloromethane afforded **3** as a white foam (1.05 g, 92%).

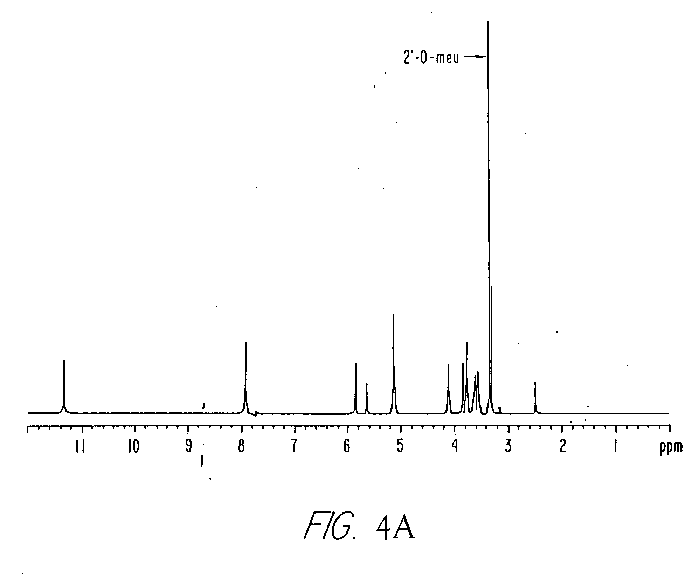

Alternately, to an oven baked stainless steel bomb (920 ml), equipped with magnetic stirrer and purged with argon, 200 ml anhydrous methanol was added followed by the addition of **2** (50 g, 0.221 mol). Trimethylborate (400 ml, 3.54 mol) was added to the resulting slurry and the bomb was sealed. The bomb was then heated in an oil bath at 130°C for 38 hours. Upon cooling, the resulting clear, slightly colored reaction mixture was evaporated *in vacuo* to afford an off white foam. Crystallization of the crude product from (methanol/ethyl acetate) gave pure 3 (56.8 g, 100%).
The identity and purity of the synthesized compound was confirmed by standard NMR analysis (Figure 4A). Following is the result of NMR analysis: ¹H NMR DMSO: 11.33 (exch. s, 1H, NH), 7.92 (d, J_{6,5}=8.2, 1H, H6), 5.85 (d, J_{1',2'}=5.2. 1H, H1'), 5.65 (d, J_{5,6}=8.2, 1H, H5), 5.13 (exch. m, 2H, 5'OH, 3'OH), 4.10 (t, J_{3',2'}=4.9, J_{3',4'}=4.6, 1H, H3'), 3.85 (m, 1H, H4'), 3.62 (dd, J_{5',4'}=3.0, J_{5',5'}=12.1. 1H, H5'), 3.54 (dd, J_{5',4'}=3.1, J_{5',5'}=12.1, 1H, H5"), 3.35 (s, 3H, OCH₃). The peak corresponding to the 2'-*O*-methyl is indicated in the figure 4A.

### Example 3: 2'-O-methyl cytidine (5)

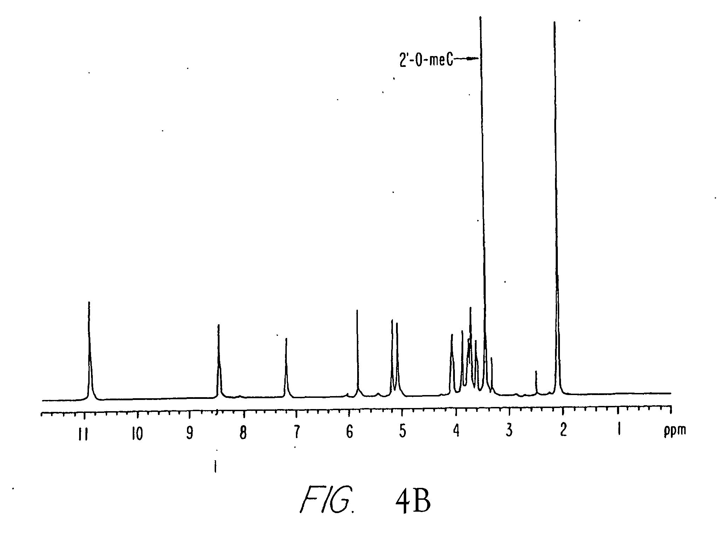

To an oven baked stainless steel bomb (300 mL), equipped with magnetic stirrer and purged with argon, 50 ml anhydrous methanol was added followed by the addition of commercially available 1g 2,2'-Anhydro-1-(β-D-arabinofuranosyl)cytosine•acetate (Aldrich) 4 (3.5 mmol). To the resulting slurry, 8 ml Trimethylborate (70 mmol) was added in the presence or absence of boron trifluoride-methanol (50%) (1.5 ml, 8.84. mmol). The bomb was sealed and then heated in an oil bath at 130°C for 38-48 hours. Upon cooling, the resulting clear, slightly colored reaction mixture was evaporated *in vacuo* to afford an off white foam. After drying *in vacuo,* the crude foam was dissolved in anhydrous DMF (50 ml) and acetic anhydride (0.36 ml, 3.85 mmol) which was added drop-wise to the reaction mixture. The resulting clear, light yellow solution was stirred overnight at room temperature. The reaction mixture was evaporated *in vacuo.* Crystallization of the crude product from (methanol/ethyl acetate) gave a pure compound 5 (0.94 g, 90%).
The identity and purity of the synthesized compound was confirmed by standard NMR analysis (Figure 4B). Following is the result of NMR analysis: ¹H NMR DMSO: 10.89 (exch. s, 1H, NH), 8.46 (d, J_{6,5}=7.4, 1H, H6), 7.18 (d, J_{5,6}=7.4, 1H, H5), 5.83 (d, J_{1',2'}=2.5, 1H, H1'), 5.18 (exch. t, J_{OH,5'}=4.6, J_{OH,5"}=4.9, 1H, 5'OH), 5.08 (exch. d, J_{OH'3},=6.7, 1H, 3'OH), 4.04 (t, J_{3',2'}=4.9, J_{3',4'}=6.8, 1H, H3'), 3.88 (m, 1H, H4'), 3.75 (dd, J_{5',4'}=2.3, J_{5',5"}=12.2, 1H, H5'), 3.59 (dd, J_{5",4'}=2.5, J_{5",5'}=12.2, 1H, H5"), 3.45 (s, 3H, OCH₃), 2.10 (s, 3H, CH₃).

2'-*O*-methyl nucleosides of the present invention can be readily converted into phosphoramidites using standard procedures and phosphoramidites can be readily incorporated into oligonucleotides, such as RNA, using standard procedures described in Sproat & Gait, 1984 in Oligonucleotide Synthesis: A Practical Approach, ed. Gait, M. J. (IRL, Oxford), pp 83-115; Usman et al., 1987 J. Am. Chem. Soc., 109, 7845; Scaringe et al., 1990 Nucleic Acids Res., 18, 5433; and Wincott et al., 1995 Nucleic Acids Res. 23, 2677-2684.

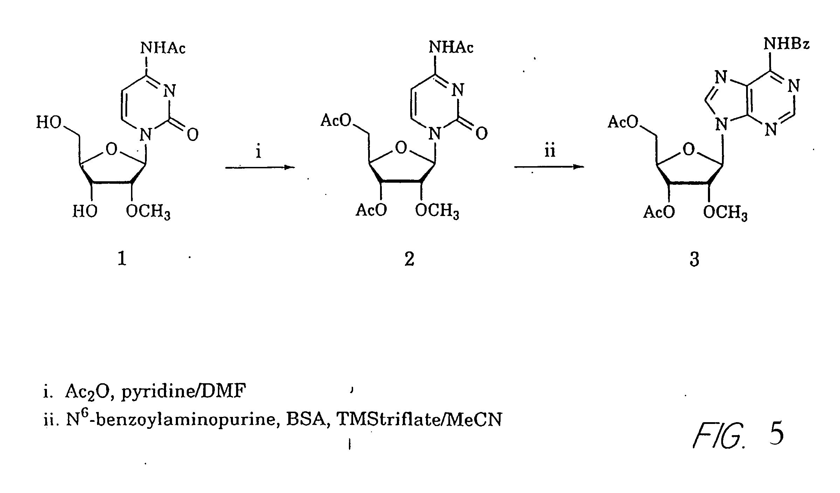

### Example 4: Synthesis of 2'-O-Me -Adenosine via transglycosilation ( Scheme 1: Figure 5)

In 1982, Imbach et al., (J. Org: Chem. 1982, 47, 202) demonstrated utilization of 2'*-O*-methyl-1,3,5-tri-*O*-benzoyl-α-D-ribofuranose in the stereospecific synthesis of 2'-*O*-methyl pyrimidine-β-D-ribonucleoside by the glycosilation of silylated bases, using Lewis acid as a catalyst. This procedure was optimized for large scale preparation of 2'-*O*-methylpyrimidine ribonucleosides by Ross et al., (J. Hetercyclic Chem. 31,765 1994). This procedure required methylation of 1,3,5-tri-O-benzoyl -α -D-ribofuranose with a large excess of potentially explosive diazomethane.

As an alternate, Applicant describes transglycosilation of suitably protected 2'-*O*-Me-pyrimidine ribonucleosides obtained through B(OMe)₃ mediated opening of 2,2'-anhydronycleosides. Transglycosylation reaction proceeds usually with high β-selectivity, if the carbohydrate donor contains a 2'-*O*-acyl group capable of stabilising the postulated C-1 carboxonium ion for exclusive β- attack by the incoming silylated base. The stereochemical outcome of transglycosilation reaction with carbohydrate donor, without the participating group at 2'-position (as 2'-*O*-Methyl) usually results in a mixture of α, and β nucleosides, as documented for the synthesis of 2'-N₃ purine ribonucleoside (Imazawa and Eckstein, 1979, J.Org. Chem. 44, 2039-41).

Applicant has investigated transglycosilation of 5',3'-di*-O*-acetyl-2'-*O-*Methyl uridine, obtained by the acylation of 2'-*O*-Methyl uridine. The transglycosilation of 5',3'-di-*O*-acetyl-2'-*O*-Methyl uridine with 3 eq of N⁵-benzoylaminopurine and 3 eq TMStriflate in CH₃CN at 75°C for 16 hour resulted in unseparable mixture of α and β isomers of *N⁶-Benzoyl-5',3'-di-O-acetyl-2'-O-methyl adenosine* in ~60% yield and 1:1 ratio. Since the nature of aglycone can also influence product distribution in transglycosilation reaction (Azuma and Isono, 1977, Chem. Pharm. Bull. 25, 3347-53), we decided to try N⁴-acetyl-5',3'-di-*O*-acetyl-2'-*O*-methyl cytidine (**2**) as a carbohydrate donor. Suprisingly, utilization of this donor under the same conditions as described above for 2'-*O*-Methyl uridine derivative resulted in the exclusive formation of β anomer of *N⁵-Benzoyl-5',3'-di-O-acetyl-2'-O-methyl adenosine* (**3**) in 50% yield. When N⁶-phenoxyacetylaminopurine was used instead of *N⁶-Benzoyl*adenine under the same conditions described above, extensive decomposition of initially formed adenosine .derivative was observed and target nucleoside was not isolated.

### Experimental Procedures for the synthesis of 2'-O-methyl adenosine and guanosine nucleosides

NMR spectra were recorded on a Varian Gemini 400 spectrometer operating at 400.075 MHz for proton and 161.947 MHz for phosphorus. Chemical shifts in ppm refer to TMS and H₃PO₄, respectively. Analytical thin-layer chromatography (TLC) was performed with Whatman MK6F silica gel 60 Å F₂₅₄ plates and column chromatography using Merck 0.040-0.063 mm Silica gel 60.

### N⁶-Benzoyl-5'3'-di-O-acetyl-2'-O-methyl adenosine (3) :

To a solution of N⁴-acetyl-2'-O-methyl cytidine(1) (1.87 g, 6.25 mmol) stirring at RT under argon in DMF/pyridine (20 ml, 20 ml) was added acetic anhydride (1.76 ml, 18.75 mmol) via syringe. The reaction mixture was stirred at RT for 18 hours then quenched with EtOH (2 ml). The reaction mixture was evaporated to dryness *in vacuo* and partitioned between dichloromethane and sat. NaHCO₃. The aqueous layer was back extracted with additional dichloromethane and the combined organics dried over Na₂SO₄. After filtration, the filtrate was evaporated *in vacuo* to afford a white foam.

A solution of N⁵-benzoylaminopurine (Lancaster) (1.23 g, 5.16_mmol) stirring in anhydrous acetonitrile under an argon atmosphere was treated with BSA (3.82 ml, 15.48 mmol) at reflux for 3 hours. Upon cooling, a solution of N⁴-acetyl-5',3'-di-O-acetyl-2'-O-methyl cytidine (**2**) (see above) (0.66 g, 1.72 mmol) in 20 ml anh. acetonitrile was added to the reaction mixture followed by TMStriflate (1.03 ml, 5.16 mmol). The reaction mixture was then heated to 75°C for 16 hours while stirring under positive pressure argon. Upon cooling, an additional 1.03 ml (5.16 mmol) of TMStriflate was added, and the reaction heated to 75°C for an additional 20 hours. Once cool, the reaction mixture was diluted with two volumes of dichloromethane and washed with sat. NaHCO₃. The organic layer was then dried over Na₂SO₄ and evaporated *in vacuo.* Flash chromatography employing a gradient of 10 to 80% ethyl acetate/hexanes afforded (1) as a white foam; 0.403 g, 50% yield. ¹H NMR (CDCl₃): 8.88 (br s, 1H, NH), 8.81 (s, 1H, H8), 8.31 (s, 1H, H2), 8.11-7.53 (m, 5H, benzoyl), 6.18 (d, J_{1',2'}=4.8, 1H, H1'), 5.41 (t, J_{3',2'}=4.8, J_{3',4'}=4.8, 1H, H3'), 4.75 (t, J_{2,1}=4.8, J_{2,3}=4.8, 1H, H2'), 4.50-4.34 (m, 3H, H4', H5', H5"), 3.44 (s, 3H, OCH₃), 2.19 (s, 3H, OAc), 2.14 (s, 3H, OAc).

## Claims

1. A process for the synthesis of a 2'-O-methyl adenosine nucleoside, comprising the step of contacting a solution of N⁴-acetyl-5',3'-di-O-acetyl-2'-O-methyl cytidine with a Lewis acid under conditions suitable for the formation of said nucleoside.

## Patentansprüche

1. Verfahren zur Synthese eines 2'-O-Methyladenosin-Nukleosides, das den Schritt aufweist: eine Lösung von N⁴-Acetyl-5',3'-di-O-acetyl-2'-O-methylcytidin mit einer Lewissäure unter Bedingungen in Kontakt bringen, die zur Bildung des Nukleosids geeignet sind.

## Revendications

1. Procédé pour la synthèse d'un 2'-O-méthyle adénosine nucléoside, comprenant l'étape de mettre en contact une solution de N⁴-acétyle-5',3'-di-O-acétyle-2'-O-méthyle cytidine avec un acide de Lewis dans des conditions appropriées à la formation dudit nucléoside.
